# EUROPEAN PATENT APPLICATION

(11) **EP 0 251 527 A2**
(43) Date of publication of application: **07.01.1988**
(21) Application number: 87305123.9
(22) Date of filing: 10.06.1987
(51) Int. Cl.: C07K 15/14, G01N 33/53, G01N 33/531, G01N 33/532, C12Q 1/68

(54) **Haptenylated reagents and immunoassays**

(30) Priority: 13.06.1986 US 874115; 10.11.1986 US 928831
(71) Applicant: Panab Laboratories, Inc., East Palo Alto CA 94303 (US)
(72) Inventor: Rice, Edward G., Palo Alto, CA 94303 (US); Derbalian, Georges P., Mountain View, CA 94043 (US)
(74) Representative: Coleiro, Raymond

(57) **Abstract**

Water-soluble avidin derivatives of the formula:

(Hapten)ₐ-A (I)

wherein Hapten is a water-soluble, non-antigenic moiety or group, preferably having a molecular weight of less than 5000, "A" is avidin, "a" is from 1 to 40 and preferably from 10 to 20, and Hapten is covalently bonded to "A" through an amino or carboxyl group thereof or through an azo linkage with a phenol group of a tyrosine or an imidazole group of a histidine group thereof, are reagent compounds for immunoassays. The preferred Haptens are water-soluble substituted aromatic groups having a quaternary amino group, arsonate group or nitro group substituent, or a ring nitrogen group.

Kits containing these compounds and antibodies specifically binding with Hapten can be used in immunoassay methods in which the compound of Formula I is contacted with a labeled antiHapten antibody for a time sufficient to permit Hapten-antibody conjugation. The label bound to the avidin derivative or the label present in the unconjugated antibody is then determined. In the preferred method of this invention, the label is an enzyme. The label is then determined by reacting a substrate with the enzyme which is selected to yield a physically measurable product, and measuring the physically measurable product produced thereby. The method can be used in nucleic acid hybridization procedures and immunoassays.

## Description

### Field of the Invention

This invention relates to improved immunoassays using biotinylated reagents. In particular, this invention relates to haptenylated avidin, labeled antihapten antibodies, and their use in biotin-avidin binding reactions in hybridization reactions and other immunoassays to provide greater amplification and sensitivity.

### Background of the Invention

A variety of processes have been developed which use biotinylated binding substances such as antibodies or nucleic acid probes for the detection and quantification of analytes in samples. In immunoassay methods using biotin, for example, insolubilized analyte can be contacted with a biotinylated binding substance, thereby attaching biotin to the analyte. Reaction of the biotin bound to the analyte with avidin bound to a detectable label or with a mixture of avidin and biotin bound to a detectable label binds the label to the analyte in a proportion which is related to the amount of analyte present. The amount of label which can be bound to each molecule of avidin is limited by surface available for binding, steric considerations and the label coupling reaction. If the label is a large molecule such as an enzyme, for example, it may be difficult to effect binding.

The analysis and detection of minute quantities of substances in biological samples has become a routine practice in clinical and analytical laboratories. DNA probes are used in a technique based on nucleic acid hybridization (polynucleotide sequence-based techniques). These are characterized by a sequence of steps comprising the non-covalent binding of a labelled polynucleotide sequence or probe to a complementary sequence of the analyte under hybridization conditions in accordance with the Watson-Crick base pairing of adenine (A) and thymidine (T), guanine (G) and cytidine (C), and the detection of that hybridization. described by M. Grunstein et al, Proc.Natl.Acad.Sci.USA. 72:3961-3965 (1975).

It is desirable to detect when binding takes place between the labelled polynucleotide sequence and the complementary sequence of the analyte. This detection is effected by determining the presence of a primary label bound to the analyte. In systems using radiolabelled binding compounds or probes, the amount of radioactivity is determined, an event which is usually directly proportional to the amount of analyte present. Amplification techniques are also employed to enhance the physically detectable signal, to yield a signal to analyte ratio which exceeds one. One amplification procedure involves selection of a primary label for which a binding partner is known, and using the binding partner as a vehicle of amplification. In a procedure embodying this invention, the primary label is biotin or a derivative thereof for which avidin is a binding partner.

### DESCRIPTION OF THE PRIOR ART

Traditional immunoassays using biotin and avidin binding are described in U.S. Patents 4,228,237, 4,434,150, 4,550,075, 4,535,057, 4,467,031 and 4,535,057, for example. In one such procedure, a sample is treated to bind the analyte (or ligand) to an insoluble phase, and the insoluble phase, separated from the sample, is reacted with a specific biotinylated binding substance for the ligand. The biotinylated binding substance which remains bound to the analyte on the insoluble phase is then reacted with enzyme labeled avidin. The enzyme remaining bound through the avidin-biotin-analyte bridge to the insoluble phase is reacted with a suitable substrate to yield a detectable enzyme-substrate reaction product. Other immunoassays using the avidin-biotin system are reviewed by Goding, J. MONOCLONAL ANTIBODIES: PRINCIPLES AND PRACTICE. New York: Academic Press pp 230-234, 244-249 (1983) and the published citations presented with reference thereto. The usual binding substance described in the above references are antibodies which selectively bind to the analyte.

Biotinylated polynucleotide probes have been developed for DNA probe applications such as the identification of unique nucleotide sequences which are characteristic of certain virus and bacteria, and in the location and identification of the presence of nucleotide sequences associated with genetic defects in human cells. Uses of biotin and dinitrophenyl labeled DNA probes are described in United States Patent 4,563,417 and European Patent Application 122,614 (Oct. 24, 1984), for example.

Signalling moieties can be any material having a detectable physical or chemical property. Such signalling moieties are described in U.S. Patent 4,563,417.

Biotinylated DNA probes and their use are described by P.R. Langer et al, Proc. Natl.Acad.Sci.USA. 78:6633-6637 (1981); J. Stavrianopoulos et al, "Glycosylated DNA Probes for Hybridization/Detection of Homologous Sequences" presented at the Third Annual Congress For Recombinant DNA Research (1983); R. Singer et al, Proc.Natl.Acad.Sci.USA. 79:7331-7335 (1982); D. Brigati et al, Virology. 126:32-50 (1983); J. Leary et al, Proc.Natl.Acad.Sci.USA. 80:4045-4049 (1983) and European Patent Application No. 63879.

Coupling a dinitrophenyl label to a phosphate group of a nucleotide in a DNA probe through a carbamoyl linkage is described in European Patent Application 119,448. Coupling biotin, iminobiotin or a dinitrophenyl group to a DNA probe through a photoactivated aryl nitrine group is disclosed in European Patent Application 155,854. Other haptens have also been used as primary labels for DNA probes, bonded through amino derivatives of adenosine or tyrosine bases as described in European Patent Application 138,597, for example. Use of N-acetoxy-N-acetyl-2-aminofluorene and related carcinogens is described by Sage et al, Biochemistry. 18:1328-1332 (1979) and in European Patent Application 158,758. In vivo labeling of probes with a glycosyl residue (which binds with Concanavalin A) or with a 5-bromodeoxyuridine group is described in European Patent Application 133,473.

Base moieties of nucleotides have been modified to create an antibody binding site label. Alkylating a nucleotide base of DNA probe to form a distinctive binding site is described in European Patent Application 128,018 and references cited therein.

A variety of compounds with binding partners other than biotin and avidin have been previously described. One or more haptens (compounds which are too small to be antigenic) have been used as substitutents on compounds and used as binding partners in hapten-antibody binding reactions in immounoassay procedures to increase the sensitivity of the procedures. United States Patents 4,185,084, 4,230,683, 4,243,749, and 4,490,473 describe haptens used in hapten-antihapten antibody sandwich immunoassays, including haptens such as the dinitrophenyl and arsonate groups, and the trimethylammonium group, alone or substituted with one or more nitro, halo, methoxy, carboxy or acetyl groups.

### SUMMARY OF THE INVENTION

The reagent compounds of this invention are water-soluble avidin derivatives of the formula:

(Hapten)ₐ-A (I)

wherein Hapten is a water-soluble, non-antigenic moiety or group, preferably having a molecular weight of less than 5000, "A" is avidin, "a" is from 1 to 40 and preferably from 10 to 20, and Hapten is covalently bonded to "A" through an amino group thereof or through an azo linkage with a phenol group of tyrosine or an imidazole group of histidine. The preferred Haptens are water-soluble substituted aromatic groups having a quaternary amino group, arsonate group or nitro group substituent, or a ring nitrogen group.

The immunoassay method of this invention includes the steps of contacting the compound of Formula I with a labeled antiHapten antibody for a time sufficient to permit Hapten-antibody conjugation. The label bound to the avidin derivative or the label present in the unconjugated antibody is then determined.

In the preferred method of this invention, the label is an enzyme. The label is then determined by reacting a substrate with the enzyme which is selected to yield a physically measurable product, and measuring the physically measurable product produced thereby.

### Detailed Description of the Invention

The reagent compounds of this invention are water-soluble avidin derivatives of the formula:

(Hapten)ₐ-A (I)

wherein
Hapten is a water-soluble, non-antigenic moiety or group, preferably having a molecular weight of from 200 to 5000,
A is avidin, and
a is from 1 to 40 and preferably from 10 to 20, and
wherein Hapten is covalently bonded to A through an amino or carboxyl group thereof or through an azo linkage with a phenol group of tyrosine or with an imidazole group of histidine.

The preferred Haptens are water-soluble substituted aromatic groups having a quaternary amino group, arsonate group or nitro group substituent, or a ring nitrogen group.

The term "avidin" is used herein to denote avidin obtained from any natural source including avidin derived from eggs, avidin derived from streptococci (strepavidin), and the like. Strepavidin is preferred because of its lower non-specific binding properties.

The haptenylated avidin reagents of Formula I can be prepared by reacting the corresponding derivatives with avidin. The reaction conditions are preferably selected to provide optimum substitution with the selected hapten moiety. Suitable hapten reactants are represented by the general formula:

(Hapten)ₐ-Y (II)

wherein
Hapten is a water-soluble, non-antigenic group, preferably having a molecular weight of less than 5000 and optimally having a molecular weight of from 200 to 5000,
Y is a halo, sulfonyl, amino or azo group, and
a is from 1 to 40 and preferably from 10 to 20.

Preferred reactants are water-soluble substituted aromatic groups having a quaternary amino group, arsonate group or nitro group substituent, or a ring nitrogen group.

One preferred reagent is represented by Formula III: 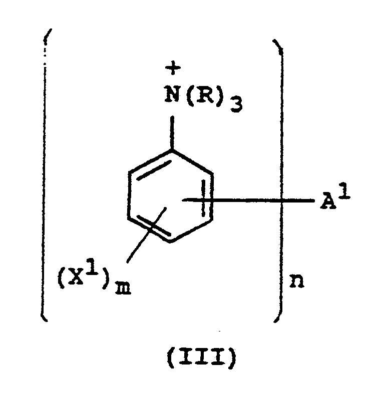 wherein
A¹ is avidin bonded through an amino or carboxyl group thereof or through an azo linkage with a phenol group of a tyrosine or an imidazole group of a histidine group thereof;
R is methyl or ethyl;
X¹ is nitro, halo (iodo, bromo, chloro or fluoro), methoxy, carboxy or acetyl;
m is 0, 1 or 2; and
n is from 1 to 40 and preferably from 10 to 20.

The compounds of Formula III can be prepared by reacting the respective compound of Formula IIIA with avidin. Formula IIIA follows: 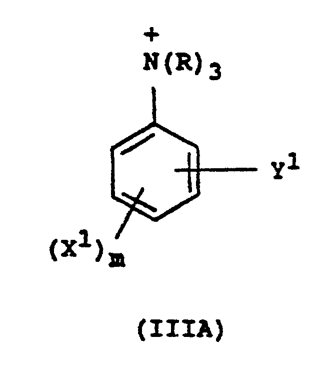 wherein
Y¹ is a iodo, bromo, chloro, fluoro, amino, diazonium salt or sulfonyl halide group, and
R, X¹ and m as are defined above with respect to Formula III.

The compounds of Formula IIIA and the method for reacting them with proteins to form linkages through the amino and other groups thereof are described in U.S. Patent 4,490,473 and the references cited therein.

Another preferred reagent is a substituted pyridine compound of Formula IV: 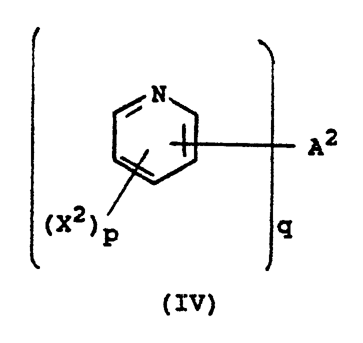 wherein
A² is avidin bonded through an amino or carboxyl group thereof, or through an azo group with a phenol group of a tyrosine or an imidazole of a histidine group thereof;
X² is nitro, halo (iodo, bromo, chloro or fluoro) methoxy, carboxy or acetyl;
p is 1 or 2; and
q is from 1 to 40 and preferably from 10 to 20.

The compounds of Formula IV can be prepared by reacting the respective compounds of Formula IVA with avidin. Formula IVA follows: 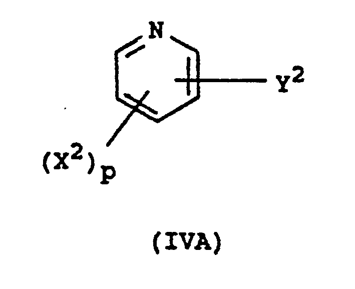 wherein
Y² is an iodo, bromo, chloro, fluoro, diazonium salt, amino or sulfonyl halide group; and
X² and p are as defined above the respect to Formula IV.

The compounds of Formula IVA and methods for reacting them with proteins to form linkages through the amino and other groups thereof are described Signor, A. et al. Nature. 205:596-597 (1965) and in commonly assigned, copending application Serial No. 873,950 filled June 13, 1986, relevant pages of which are filed with this application.

Another preferred reagent is a nitrophenyl compound of Formula V: 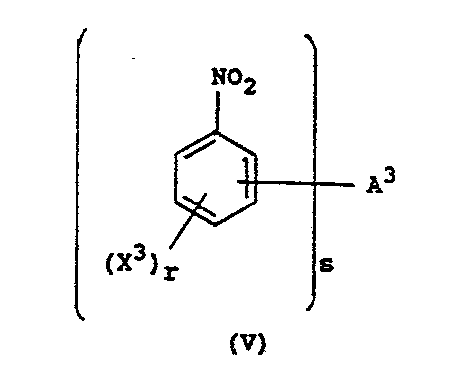 wherein
A³ is avidin bonded through an amino or carboxyl group thereof, or through an azo linkage with a phenol group of tyrosine or with an imidazole group of histidine thereof;
X³ is nitro, halo (iodo, bromo, chloro or fluoro) methoxy, carboxy or acetyl;
r is 1 or 2; and
s is from 1 to 40 and preferably from 10 to 20.

The compounds of Formula V can be prepared by reacting the respective compound of Formula VA with avidin. Formula VA follows: 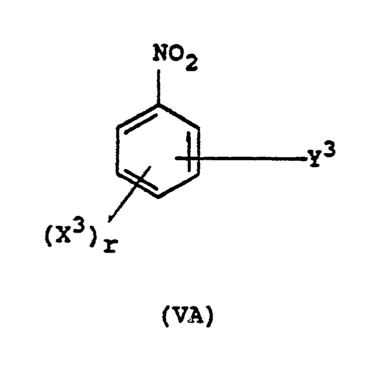 wherein
Y³ is an iodo, bromo, chloro, fluoro, amino, diazonium salt or sulfonyl halide group; and
X³ and r are as defined above regarding Formula V.

Compounds of Formula VA are well known in the art. For example halonitrobenzene compounds are described with methods for their preparation by Hodgson, H et al, J.Chem.Soc. 545:819 (1945), Miyajima, G. et al, Chem.Pharm.Bull. 19:2301 (1971), Olah, G. et al, J.Org.Chem. 43:4628 (1978), Holleman, A. et al, Recl.Trav.Chim.Pays-Bas 35:46 (1915, Tallec, A. Am.Chem.(Paris) 3:164 (1968) and Welsh, L. J.Am.Chem.Soc. 63:3276 (1949).

A still further preferred reagent is an arsonate represented by Formula VI: 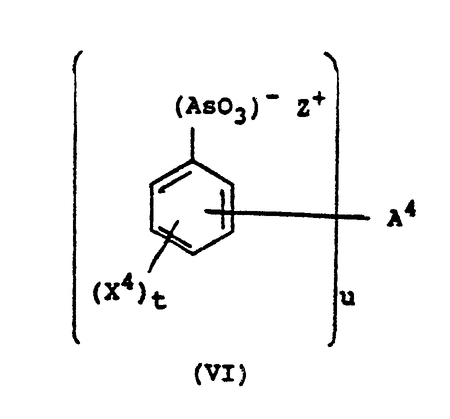 wherein
A⁴ is avidin bonded through an azo linkage with the phenol group of tyrosine or with an imidazole group of histidine group thereof;
X⁴ is nitro, halo (iodo, bromo, chloro or fluoro) methoxy, carboxy or acetyl;
Z is an alkali metal, alkaline earth metal, ammonium group, or quaternary lower alkyl amino group;
t is 0, 1 or 2; and
u is from 1 to 40 and preferably from 10 to 20.

The compounds of Formula VI can be prepared by reacting the respective compound of Formula VIA with avidin. Formula VIA follows: 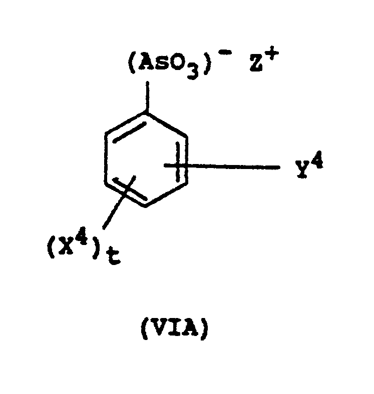 wherein
Y⁴ is an diazonium salt group; and
X⁴, Z and t are as defined above the respect to Formula VI.

The compounds of Formula VIA and methods for reacting them with proteins to form linkages through the azo groups are described by Alkan, S. et al, The Journal of Immunology. 107(2):353-358 (1971), Alkan, S. et al, The Journal of Experimental Medicine. 135:1228-1246 (1972) and Goodman, J. et al, Immunol.Review. 39:35 (1978).

In general, the avidin is haptenated by reacting a compound of Formula II with avidin in an aqueous solution. For hapten reactants wherein the linking moiety is a halo or sulfonyl group, the reaction solution can be completely aqueous or certain polar, water-miscible solvents such as acetone or dimethyl sulfoxide can be present. The reaction solution has a pH of from 2 to 12, preferably from 6.5 to 10.5, and optimally from 9 to 9.5. Buffering salts such as phosphate, borate, carbonate or organic buffering ions which are not themselves reactive with either reactant can be present. The reaction is preferably carried out at a temperature of from 0 to 60°C and optimally at a temperature of from 15 to 45°C. The time required for completing the reaction is temperature dependent, a reaction time of at least 5 minutes usually being sufficient at temperatures of from 15 to 45°C.

When the linking moiety is an amino group to be reacted with a carboxyl group of avidin to form an amide linkage, the reaction can be carried out with a carbodiimide according to the procedures disclosed in U.S. Patents 3,825,525 and 4,486,344, and Hanna, N. et al, Proc.Soc.Exp.Biol.Med. 104(1)89-92 (1972), the entire contents of which are hereby incorporated by reference in their entireties. The reaction can also be carried out with a carbonyl diimidazole as described in U.S. Patent 4,486,344, THE PEPTIDES, Vol. 1, E. Gross and J. Meienhofer, Eds. New York: Academic Press, p 66 (1979), and Axen. U., Prostaglandins. 5(1):45-47 (1974), the entire contents of which are hereby incorporated by reference in their entireties. This reaction is carried out in aqueous solution having a pH within the range of from 6 to 9 and preferably from 7 to 8. Standard carbodiimides can be used such as
1-ethyl-3-(-N,N-dimethylaminopropyl)carbodiimide or 1-cyclohexyl-3-(2-morpholinoethyl)carbodiimide methyl-p-toluenesulfonate. Other reactants which can be used include difunctional aldehydes such as glutaraldehyde.

The haptenylated avidins of this invention are useful in immunoassays wherein they are conjugated with antibodies which selectively conjugate with the respective hapten. These antibodies are denoted herein as antiHapten antibodies, and suitable antiHapten antibodies of use in accordance with this invention can be either polyclonal and monoclonal antibodies, or mixtures thereof. Methods for preparing antibodies which selectively bind to haptens are well known in the art and are not part of this invention. The antibodies can be obtained in any available manner such as conventional antiserum and monoclonal techniques. Antiserum can be obtained by well-established techniques involving immunization of an animal, such as a mouse, rabbit, guinea pig or goat, with the hapten bound or complexed with an antigenic protein such as a serum albumin such as bovine serum albumin (BSA), human serum albumin (HSA), and the like, keyhole limpet hemocyanin (KLH), or any other antigenic protein which will produce a desirable antibody titer. The antibodies can alternatively be obtained by somatic cell hybridization techniques, to yield monoclonal antibodies.

Polyclonal antibodies are preferably purified to remove antibodies which bind to the antigenic portion of hapten-antigen complex or compound rather than the hapten portion. This purification can be effected by conventional procedures which are not a part of this invention such as conventional affinity chromatography of the animal serum containing the antibodies or an antibody fraction obtained therefrom. In this procedure, the antibody solution is passed through a column containing the hapten conjugated to an insoluble support such as SEPHAROSE beads (Pharmacia), and the antiHapten antibody bound and retained by the insoluble support is selectively eluted therefrom.

The antiHapten antibody can be labeled with a suitable detectable label or chemical group for use in accordance with this invention. Such a label can be any material having a detectable physical or chemical property. Such labels are described in U.S. Patent 4,563,417 which is hereby incorporated by reference in its entirety. Such materials have been well-developed in the field of immunoassays, and in general, most any label useful in such methods can be applied to the present invention. Particularly useful labels are enzymatically active groups, such as enzymes described in Clin.Chem. 25:353 (1979), enzyme substrates such as are described in British Patent Application No. 1,548,741, coenzymes such as are disclosed in U.S. Patents 4,230,797 and 4,238,565, enzyme inhibitors as are described in U.S. Patent 4,134,792, fluorescers described in Clin.Chem.. 25:353 (1979), luminescers such as chemiluminescers and bioluminescers described in Clin.Chem.. 25:512,1531 (1979), specifically bindable ligands, proximal interacting pairs; spin labels, and radioisotopes such as ³H, ³⁵S, ³²P, ¹²⁵I and ¹⁴C. Such labels and labeling pairs are detected on the basis of their own physical properties (e.g., fluorescers, chromophores and radioisotopes) or their reactive or binding properties (e.g., enzymes, substrates, coenzymes and inhibitors). For example, a cofactor-labeled antibody can be detected by adding the enzyme for which the label is a cofactor and substrate for the enzyme. A hapten or ligand labeled antibody can be detected by adding an antibody to the hapten or a protein which will bind with the ligand (biotin to avidin), tagged with a detectable molecule. Such a detectable molecule can be some molecule with a measurable physical property (e.g., fluorescence or absorbance) or a participant in an enzyme reaction such as described above.

For example, one can use an enzyme which acts upon a substrate to generate a product with a measurable physical property. Examples of the latter include, but are not limited to, β-galactosidase, alkaline phosphatase and horse radish peroxidase. Ideally, the final product is water-soluble. Proximal interacting or linking labels as are well known in the immunoassay field are described in Clin.Chem.. 27:1797 (1981) and U.S. Patents 3,996,345 and 4,223,402 can be applied to the present method by using two different populations of antibodies, one labeled with one member of a pair and the other labeled with the other member of the pair. For instance, a first portion of antiHapten antibodies is labeled with a fluorescer and a second portion is labeled with a quencher. The presence of the hapten is indicated by quenching of the fluorescence due to the proximate binding of the first and second portion antibodies along the haptenated avidin. Similarly, one can use first and second enzyme labels where the product of one is a substrate for the other. The presence of complexes is then indicated by increased turnover of the second enzyme due to the proximate enzyme channeling effect. Other labeling schemes will be evident to one of ordinary skill in the art.

Alternatively, the antiHapten antibody can be detected based on a native property of the antibody such as its own antigenicity. A labeled anti-(antibody) antibody (secondary antibody) will bind to the primary antibody where the label for the secondary antibody is any conventional label as described above. Further, antibody can be detected by complement fixation or the use of labeled protein A, as well as other techniques known in the art for detecting antibodies.

When the antiHapten antibody is labeled, as is preferred, the labeling moiety and the antibody reagent are associated or linked to one another by direct linkage such as involving covalent bonds or other binding, or by indirect chemical linkage such as by incorporation of the label in a microcapsule or liposome which is in turn linked to the antibody. Labeling techniques are well-known in the art and any conventional method can be used in the present invention.

The immunoassay method of this invention includes the steps of contacting a water-soluble avidin derivative of Formula I with a labeled antiHapten antibody for a time sufficient to permit Hapten-antibody conjugation. Formula I follows

(Hapten)ₐ-A (I)

wherein
Hapten is a water-soluble, non-antigenic group, preferably having a molecular weight of from 200 to 5000, a is from 1 to 40 inclusive,
A is avidin, and
wherein Hapten is covalently bonded to A through an amino or carboxyl group thereof or through an azo group with a phenol group of a tyrosine or an imidazole group of a histidine group thereof.

The label bound to the avidin derivative or the label present in the unconjugated antibody is then determined.

The method of this invention is most advantageously applied to the detection of the presence of the binding partner of the avidin-biotin binding pair in an immunoassay. A variety of immunoassays using avidin and biotin, and the binding interaction therebetween, are described hereinabove in the background of the invention.

The term "nucleic acid probes", as used herein, includes both DNA and RNA probes. The method of this invention is described primarily in terms of DNA probes, for purposes of clarity and not by way of limitation, and all types of nucleic acid probes are intended to be included.

The method of this invention can be used in any immunoassay wherein a biotinylated reagent is conjugated with another compound or material. Biotinylated nucleic acid probes are described in U.S Patent 4,563,417, British Patent No. 2,019,408 and European Patent Application No. 63,879. Use of avidin labeled reagents which bind specifically to a ligand in an immunoassay for the ligand is described in U.S. Patents 4,228,237, 4,550,075 and 4,535,057, for example.

The biotinylated nucleic acid probes used in the method of this invention can be coupled to nucleotides in the nucleic acid probe or a oligonucleotide sequence coupled thereto, through a nucleotide base or phosphate group. This coupling can be achieved in a variety of ways. In one coupling procedure, the biotin is derived from a biotin aryl azide precursor. Aryl azides are stable in the dark and can be photoactivated in situ to generate highly reactive aryl nitrines. Nitrophenyl azides are preferred because they can be photoactivated with visible light, avoiding irradiation with ultra-violet light which might damage the nucleic acid probe.

Suitable biotin aryl azide precursors can be represented by formula VII. These compounds and methods for their preparation and use to prepare biotinylated nucleic acid probes are described in European Patent Application 155,854, the entire contents of which are hereby incorporated by reference.

Biotin-(B)ₐ-N₃ (VII)

wherein
B is a nitroaryl phenyl linking group substituted with an alkyl or halo-substituted alkyl group having from 1 to 12 carbons or a solubilizing hydrocarbon amine residue containing at least 5 carbon; and
a is 0 or 1.

The compounds of Formula VII wherein the linking groups (B) are nitroaryl phenyl linking groups are represented by Formula VIII: 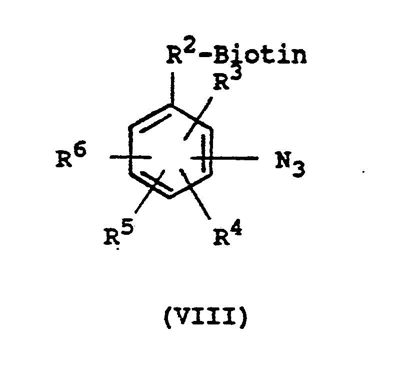 wherein
R² is a solubilizing hydrocarbon amine residue having at least 5 carbons, preferably a compound of Formula IX, bonded to the biotin through an amide linkage with the alkyl carboxylic acid group of biotin; and
R³, R⁴, R⁵ and R⁶ are the same or different, are each hydrogen, halo, carboxylic acid, amino or an acid addition salt thereof.
Formula IX follows:

-(CHR⁷)_{b}-N(R⁸)_{c}-(CHR⁹)_{d}-(R¹⁰)ₑ-NH- (IX)

wherein
R¹⁰ is -NH-CO-CHR¹¹- or -NH-(CHR¹²)_{f}-;
R⁷, R⁸, R⁹, R¹¹ and R¹² are the same or different, are each hydrogen, halo or alkyl having from 1 to 5 carbons;
b, d and f are the same or different, are each independently an integer of from 1 to 10;
c is 0 or 1; and
e is 0 or an integer of from 1 to 5.

Preferred examples of hydrocarbon amine residues of Formula IX are:

-(CH₂)₃-N(CH₃)-(CH₂)₃-NH-

-(CH₂)₃-N(CH₃)-(CH₂)₃-NH-CO-CH₂-NH-

-(CH₂)₃-N(CH₃)-(CH₂)₃-(NH-CO-CH₂)₂-NH-

-(CH₂)₃-NH-(CH₂)₄-NH-

-(CH₂)₃-NH(CH₂)₄-NH-(CH₂)₃-NH-

The compounds of Formula V wherein (a) is 1 can be made by reacting the succinimide ester of biotin with the diamine of Formula IX.

Another biotinylated nucleic acid probe has the biotin coupled to the probe through a phosphate group. These compounds and methods for their preparation and use in hybridization are described in European Patent Application 119,448, the entire contents of which are included herein in their entirety. In this coupling procedure, the biotin can be coupled to an amino group which is coupled to terminal phosphate moiety of a nucleic acid probe to yield a compound of Formula X. 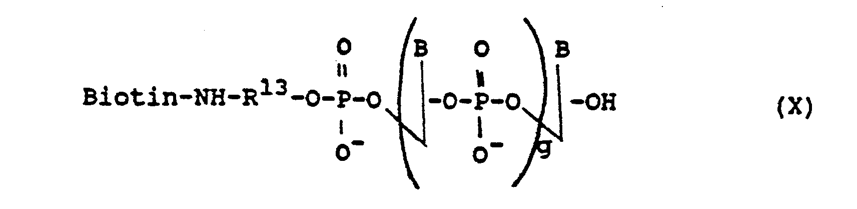 wherein
B is a deoxyribonucleoside residue after removal of 3ʹ and 5ʹ-hydroxyl groups from 2ʹ-deoxyribonucleoside; and
R¹³ is a straight or branched chain hydrocarbon residue, preferably having from 1 to 24 carbons, an optimally having from 1 to 12 carbons;
g is a integer, preferably from 7 to 100.

The compounds of Formula X can be prepared by reacting the corresponding amines of Formula XA with the carboxylic acid group of biotin to form an amide linkage. 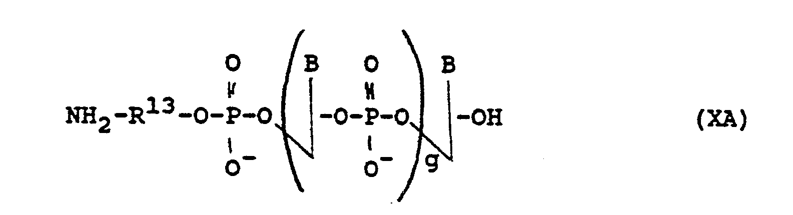 wherein R¹³ and g are as defined with respect to Formula X.

The compounds of Formula XA can be prepared by condensing the corresponding oligonucleotide with a corresponding aminoalkanol compound of which the amino group is protected, for example with a trifluoroacetyl group according to the procedures in European Patent Application 119,448 and Japanese Patent Application 138,136/1982, the entire contents of which are hereby incorporated by reference in their entireties.

The oligonucleotide probes can be derived from natural materials or can be synthesized by conventional procedures well known in the art and which are not a part of this invention. In general, they can be synthesized by the triester method and the phosphite method, each involving the solid phase method and the liquid phase method. Suitable procedures are described in Tetrahedron Letters. 1979:3635 (1979), Nucleic Acids Research. 8:5473, 5491, 5507 (1980) and Nucleic Acids Research Symposium Series. 7:281 (1980).

The compounds of Formula V wherein (a) is 1 can be made by coupling biotin through a linking group with the primary amino groups of adenine or cytosine. This can be effected following the procedure described in European Patent Application 138,357, the entire contents of which are hereby incorporated by reference in its entirety.

The biotin labeling is accomplished by reacting the nucleic acid probe containing adenine and/or cytosine with a reagent of Formula XI. 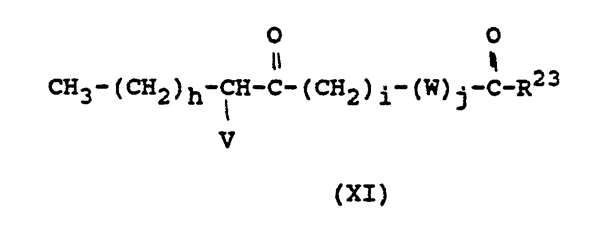 wherein
R²³ is the residue of the biotin molecule;
V is halo, including fluoro, chloro, bromo and iodo;
W is -SO₂-(CH₂)ₖ-
h is 0 or an integer from 1 to 9;
i is an integer of from 1 to 10 and preferably from 1 to 4;
j is 0 or 1; and
k is an integer from 1 to 10.

The compounds of Formula XI wherein j is 1 are made by the procedure of Fink et al, Anal.Biochem. 108:394-401 (1980). To make the compounds wherein j is 0, a modified Fink et al reaction is carried out. Equimolar amounts of the desired keto-acid or keto-ester are mixed with molecular halogen V₂ (preferably bromine) in chloroform. After consumption of the halogen, the alpha-haloketo-acid or ester can be used without purification, or may be further purified, using standard procedures.

Generally, the reaction between the linking group of Formula XI and the oligonucleotide probe is carried out for about one hour at about 37°C, and excess linking reagent is removed, for example by extraction with an organic solvent. Adenine and cytosine react with the linking reagent to form, after dehydration, a stable derivative. The resulting derivatized probe is coupled to the carboxylic acid group of biotin through a group, reactive therewith. The N-hydroxysuccinimide ester of biotin can be used to couple with an amine group, for example. Primary amines can react with the acyl carbon atom of the ester group to form an amide bond. This reaction can be carried out at pH 7 to 8 in a buffer, e.g. sodium bicarbonate. The reactants are incubated together for a time sufficient to permit the reaction to proceed to completion, usually from 4-20 hours, at a suitable temperature, usually room temperature. The final product is purified, e.g. by gel purification.

The oligonucleotide probe can be made with a polyadenine, polycytosine or adenine-cytosine copolymer tail which can be labeled with hapten with a linking compound of Formula XI. The degree of hapten substitution can be controlled by the relative proportions of reactants.

In an alternate procedure, the biotin can be linked to the pyrimidine base of uridine triphosphate through an allylamine linker arm, and the product can be used as a substrate for probe synthesis using DNA and RNA polymerases in vitro, following a modified procedure of P.Langer et al, Biochemistry. 78(11):6633-6637 (1981), the entire contents of which are hereby incorporated by reference in their entireties. The 5-mercurated derivatives of uridine or deoxyuridine are prepared by a modification of the procedure of Dale et al, Biochemistry. 14:2447-2457 (1975). The 5-(3-amino)allyluridine and deoxyuridine 5ʹ-triphosphates are prepared by reaction with the olefin in the presence of a palladium catalyst.

The N-hydroxysuccinimide ester of biotin is prepared from biotin by conventional procedures. The allylamine substituted compound and the hapten succinimide ester are reacted at room temperature in DMF for 4 hours, and the reaction mixture purified by column chromatography.

The biotinylated uridine triphosphate or deoxyuridine triphosphate can function as substrates for a series of purified nucleic acid polymerases in vitro. Examples of these are the DNA polymerase I using either the nick-translation protocol of Rigby et al (19) or the "gap-filling" reaction described by Bourguignon (21), bacteriophage T4 polymerase, DNA polymerases alpha and beta from murine and human (HeLa) cells, DNA polymerase of herpes simplex virus. The ribonucleotide analog, haptenylated uridine triphosphate can substitute for uridine triphosphate in reactions catalyzed by the RNA polymerases of E. coli and bacteriophage T7.

Nucleic acid probes can be terminally labeled with the haptenylated uridine and deoxyuridine triphosphate using a terminal transferase such as terminal deoxynucleotidyl transferase in accordance with the procedures of European Patent Application 122,614, the entire contents of which are hereby incorporated by reference in their entireties.

The biotinylated probes can also be made by coupling the biotin with 8-aminohexyl adenosine 5ʹ-triphosphate in accordance with the procedures described by Vincent et al, Nucleic Acids Research. Vol. 10, No. 21, Oxford, England: IRl Press (1982), the entire contents of which are hereby incorporated by reference in its entirety. The biotinylated adenosine can be incorporated into a probe using terminal deoxynucleotidyl transferase or other enzymes. Alternatively, the 8-aminohexyl adenosine 5ʹ-triphosphate can be incorporated into the probe, and the biotinylating compound can be conjugated with the probe thereafter.

The haptenylated probes can be used in a number of assay procedures. The method of this invention includes the steps of contacting a biotinylated nucleic acid probe with a nucleotide sequence, and determining the presence and/or quantity of hybridized probe which has coupled with the nucleotide sequence. The hybridized probe is determined by contacting the sample having hybridized probe bound thereto with a haptenylated avidin, and conjugating the hapten with an antibody which binds specifically with the hapten. The antibody can be conjugated with a physically distinguishable secondary label such as a radiolabel or chromophore, or an enzyme which will generate a physically distinguishable product when contacted with a suitable substrate. These procedures can be applied in a variety of methods.

The reagents of this invention can be used in all of the standard quantitative hybridization methods to provide improved sensitivity. These methods are summarized in detail by Walker, J. in METHODS IN MOLECULAR BIOLOGY: VOLUME 2 NUCLEIC ACIDS. Clifton, NJ: Humana Press (1984); NUCLEIC ACID HYBRIDIZATION: A PRACTICAL APPROACH. (B. Hames and S. Higgins, eds), Oxford: IRL Press (1985) and Singer, R. et al, BioTechniques. 4(3):230-250 (1986), the entire contents of each of these references and the references cited therein being hereby incorporated by reference in their entireties. Hybridizing methods are generally characterized as solution hybridization, filter hybridization and in situ hybridization. Filter and in situ hybridization methods include most quantitative and qualitative techniques using labeled probes.

In filter hybridization, denatured DNA or RNA is immobilized on an inert support such as nitrocellulose or another polymer with similar binding properties. The binding is effected in such as way that self-annealing is prevented but bound sequences are available for hybridization with an added nucleic acid probe. To facilitate analysis according to this invention, the probe is labeled with biotin or a biotin derivative. Detection of hybrids is usually by autoradiography if the secondary label is radioactive, although if the hybrids are sufficiently radioactive, a scintillation counter can be used. The filter hybridization technique is widely applicable, being used for phage plaque and bacterial colony hybridization, Southern and Northern blot hybridization, dot blot hybridization and hybrid selection.

Dot blot hybridization is well suited to the analysis of multiple samples. It has the added advantage that it is easy to prepare replicate filters allowing many filter-bound sequences to be analysed at the same time, for example with different probes or under different hybridization and washing conditions. Dot blot hybridization can be used qualitatively since it is capable of great discrimination. It can also be used quantitatively with appropriate calibration. The limits of this method are low rates of hybridization and sensitivity.

The filter hybridization applications of the biotinylated probes of this invention are described herein in terms of a standard procedure for purposes of clarity and not by way of limitation, for the probes can be used in any probe hybridization technique wherein the hybridized probes are to be determined.

A wide variety of filter materials are available for immobilization of DNA and RNA, for example, nitrocellulose, nylon and chemically activated papers. All filters require the nucleic acid to be denatured for binding.

In the DNA dot blot procedure, multiple samples of genomic or plasmid DNA are spotted next to each other on a single filter in dots of uniform diameter. For quantitative analysis, known amounts of DNA are applied. To evaluate the extent of hybridization of the probe, a standard consisting of a dilution series of DNA dots is applied in an identical way to the same filter. Since the samples bind quickly, they can be handled immediately. Dot blots do not distinguish the number or size of the molecules hybridizing, so the hybridization signal is the sum of all sequences hybridizing to the probe under the conditions used. The procedures vary only slightly when binding plasmid DNA, genomic DNA and RNA.

It is important to characterize the nucleic acid used for the probe. Repetitive elements must be removed if the probe is to be used to detect low copy number sequences, to avoid making of the desired hybridization with repetitive sequence hybridization. The length of the labelled probe is important since the kinetics of hybridizition depend on probe length. The kinetics of hybridization differ according to whether the probe or filter-bound sequences are in excess. These factors can be determined by varying the amount of probe; if the filter-bound sequences is in excess, the mount of hybridization is proportional to the probe input. Vary the amount of nucleic acid on the filter; if in excess, there should be no difference in the amount of probe hybridized.

The hybridization process can be divided into three steps, pre-hybridization, hybridization and washing. In the pre-hybridization step the filter is incubated in a solution which is designed to pre-coat all the sites on it which bind the probe non-specifically. Failure to do this leads to high backgrounds. Usual pre-hybridization solutions contain Ficoll, polyvinyl pyrollidone and bovine serum albumin (Denhardt's solution), and heterologous DNA. As an alternative, heparin can substitute for Denhardt's solution. To reduce backgrounds even further, poly(A) and poly(C) are often included. Poly(A) is useful when the probe or filter-bound sequences are rich in A and T residues, e.g., poly(A)⁺ mRNA or cDNA derived from it. Similarly, poly(C) is included if the probe or filter-bound sequences are rich in G and C residues as when the recombinant is generated through oligo(dG) and oligo(dC) homopolymer tailing. For hybridizations involving RNA, yeast tRNA is often used as a competitor.

For hybridization, it is necessary to ensure that the added nucleic acid is single-stranded. For double-stranded DNA probes, this is usually achieved by boiling or by denaturing in alkali. Alkali should be avoided if it would degrade the linkage bonds in the probe, i.e., amide linkages. For most purposes hybridization can be carried out either in aqueous solution or in the presence of formamide. Formamide is preferred if elevated temperatures will degrade the probe. Both RNA-DNA and DNA-DNA hybridizations can be carried out in formamide, but DNA-DNA hybridizations, only, should be carried out in aqueous solutions. Both protocols can be used with nitrocellulose and nylon filters.

After hybridization, washing is carried out to remove unhybridized probe and to dissociate unstable hybrids. The temperature and salt concentration of the washing solution determine which hybrids will be dissociated. In general, washing should be under as stringent conditions as possible; at 5-20°C below the melting temperature (Tₘ) for well-matched hybrids and 12-20°C below T^{m} for cross-hybridizing probes. The preferred ranges are 65-70°C for hybrids having a high degree of homology and 50-60°C for poorly matched hybrids.

The pre-hybridization, hybridization and washing steps should be carried out in a shaking water bath or on a shaking platform in an incubator. In filter-bound nucleic acid excess, diffusion of the probe to the filter can be limiting in the absence of agitation. Also, high backgrounds are sometimes encountered if there is no shaking. Solutions should be pre-warmed to the required temperatuare before use.

The probe is then detected by conjugating the labeled probe bound to the filter with haptenylated avidin and an anti-(hapten label) antibody, and the antibody binding to the filter is determined.

The probes can also be used in hybridizations in situ within cytological preparations, permitting spatial localization of sequences complementary to the probe. The probes can be used in two types of in situ hybridizations, hybridization to nuclear DNA and hybridization to cellular DNA. These hybridization methods are general similar, differing only in specific details which are generally known in the art and are not a part of this invention. These procedures are carried out with subbed slides which give better retention of the cytological preparations. The slides are thoroughly cleaned with acid or in detergent and thoroughly rinsed in water. They are put into a final rinse of distilled water and then dipped into a subbing solution. This is an aqueous solution of 0.1% gelatin and 0.01% chrom alum [CrK(SO₄)₂· 12 H₂O]. The slides are then dried. The cover slips are siliconized to prevent adherence of cytological materials. Incubations are carried out in moist chambers to prevent evaporation from the cytological preparations during incubation.

The procedure for preparing samples for in situ hybridization differs for the cytological preparations. Metaphase and prometaphase chromosome spreads are prepared from aminopterin-synchronized cell cultures. The cells are separated from media, washed, fixed with freshly-made methanol:acetic acid (3:1 v/v), incubated for 20 min, centrifuged, resuspended in fixative, recentrifuged, repeating this cycle several times, and then applied to the slide. Tissue samples are minced in fixative, applied to the slide surface in a thin pressed layer (maximum thinness), frozen in dry ice, and dehydrated in ethanol washes.

The slides are then pretreated to denature the DNA prior to exposure to the probe. All of the reagents which denature purified DNA, such as alkali, heat, and organic solvents, also denature DNA in cytological preparations and can be used in in situ hybridization Acid denatured preparations give a reduced amount of in situ hybridization and are not preferred.

The parameters affecting in situ hybridization are similar to those applicable for hybridizing DNA fixed on nitrocellulose filters. Conditions of ionic strength, temperature, probe concentration and hybridization time are chosen using the same criterion. The conditions chosen determine the stringency of the hybridization reaction and set limits on the amount of sequence mismatching that can occur. In situ hybridization can be carried out either in salt solution at high temperature or in formamide solution at lower temperature, the choice being determined by the thermal stability of the probe. Dextran sulfate can be used to accelerate the hybridization.

Most hybridizations to cellular DNA required only the preservation of chromosome or, at most, nuclear morphology. In contrast, studies of the localization of RNA in cytological preparations require preservation of the morphology of the entire cell, and in some cases, of tissues and organs. Therefore, techniques used are more cell type-specific than the techniques used for DNA studies. A review of these procedures and examples of variations are provided by Pardue, M. "In situ hybridization," NUCLEIC ACID HYBRIDIZATION (supra, pp 179-202), hereby incorporated by reference,

In sandwich immunoassays, for example, the avidin reagent is conjugated (caused to bind) with ligand which has been previously insolubilized on an insoluble support. Removal of the unbound avidin reagent from the insoluble support leaves the bound avidin, bound to the ligand. Determination of the avidin bound to the insoluble support has traditionally been accomplished by conjugating the avidin on the insoluble support with the respective avidin or biotin binding partner.

In accordance with the improved method of this invention, the insolubilized biotinylated reagent can be determined by conjugating or causing it to bind with the corresponding haptenylated (hapten conjugated) avidin or biotin, respectively. The insolubilized support to which haptenylated reagent is bound can be conjugated with a suitably labeled, antiHapten antibody, and the label determined to yield the presence or concentration of the target ligand.

In an immunoassay for determining the presence of a ligand in a solution sample, for example, the sample can be incubated with an insoluble support to which an anti-ligand antibody has been adhered. The contact or incubation time should be sufficient to permit substantial conjugation to occur between the ligand and the anti-ligand antibody, the time being temperature dependent. Suitable incubation times are from 2 to 180 minutes at temperatures of from 18 to 40°C, the preferred contact time being within the range of from 2 to 120 minutes at temperatures within the range of from 20 to 26°C.

The sample solution is then removed from the insoluble support and rinsed with a suitable buffer solution such as is described in U.S. Patent 4,528.267. Such a rinse solution can have a pH of from 6 to 8 and can be a phosphate buffer solution having a phosphate molarity of from 0.01 to 0.05 and containing from 0.01 to 0.1 weight percent non-ionic surfactant. It can have from 0 to 1.8 weight percent of non-interfering animal protein.

The insoluble support is then incubated with a biotinylated anti-ligand antibody for a time sufficient to permit conjugation between the anti-ligand antibody and any ligand on the insoluble support. Suitable incubation times are from 30 to 180 minutes at temperatures of from 4 to 40°C, the preferred contact time being within the range of from 2 to 120 minutes at temperatures within the range of from 20 to 26°C. The insoluble support is then rinsed with a suitable rinse solution such as described above to remove any unconjugated anti-ligand antibody.

The biotin bound to the insoluble support can then be determined by incubating it with a solution containing haptenylated avidin for a time sufficient to permit avidin-biotin binding to occur. The haptenylated avidin solution can have from 0.1 to 1.0 mg/ml of haptenylated avidin and a pH of from 5 to 10.5. The insoluble support is then rinsed with the rinse solution to remove any unconjugated haptenylated avidin.

The support is then incubated with an antiHapten antibody solution. This solution can contain from 0.1 micrograms/ml to 1 mg/ml of the antibody and be incubated for the time and temperatures described above for antibody-ligand reactions. The unconjugated antiHapten antibody is then removed, the insoluble support rinsed, and the antiHapten antibody bound to the insoluble support is determined.

In a preferred embodiment of the method of this invention, the antiHapten antibody is labeled with an enzyme label. The enzyme label bound to the insoluble support following the above sequence is determined by contacting the insoluble support with a solution of a substrate which undergoes chemical reaction in the presence of the enzyme to produce a physically measurable product such as a chromophore or a fluorophore. Suitable substrates and enzymes they are converted by are known in the art and are described in U.S. Patent 4,190,496, for example.

The solid support is contacted with an aqueous solution containing from 10⁻² to 10⁻¹⁰ molar concentrations of the substrate for a time sufficient for generation of a measurable amount of the enzyme reaction product. At temperatures of from 19 to 40°C, incubation times of from 2 to 240 minutes can be used. The level of the enzyme reaction product in the substrate solution then measured by suitable means appropriate for the selected enzyme and substrate, and compared with controls to yield a concentration of ligand in the original sample.

Similar procedures can be applied to procedures using biotinylated DNA probes. A variety of procedures yielding insolubilized biotinylated probes hybridized with DNA are described in U.S. Patent 4,563,417. In one approach, single stranded nucleic acids from the test medium are first immobilized on a solid support before hybridization with a labeled probe. In alternative procedures, the nucleic acid strands are reacted with the biotinylated DNA probed in solution before solubilization and binding to an insoluble support.

The products of this invention can be the individual reagents described above or kits containing various combinations of the reagents. For example, kits containing biotinylated nucleic acid probes or biotinylated antibodies; haptenated avidin; labelled or unlabeled antibodies binding specifically with the hapten bound to the avidin or combinations of these reagents.

This invention is further illustrated by the following specific but non-limiting examples wherein completed laboratory procedures which have been performed are presented in the past tense and laboratory procedures which are reduced to practice herein are presented in the present tense. Temperatures are given in degrees Centigrade and percents as weight percents unless otherwise specified.

### EXAMPLE 1

### Haptenylated Streptavidin

To each of reaction tubes #1 and #2 was added 1000 ul (microliters) of a 2 mg/ml solution of streptavidin (Sigma, S4762) in PBS, pH 7.4; 400 ul of 1 M NaHCO₃ solution, pH 9.5; and 400 ul of a solution of 20 mg/ml of 4-fluoro-3-nitrotirmethylphenyl ammonium iodide (PAN-F reagent, PanAb Laboratories, East Palo Alto, CA) in deionized water. Each tube was incubated in a dry-air incubator maintained at 37°C. Tube #1 (F-SA#1) was incubated for 2 hr, and tube #2 (F-SA#2) was incubated for 3 hr. The contents of each reaction tube was placed in a separate dialysis membrane with a nominal molecular weight exclusion limit of 1000 daltons. Each reaction mixture was dialyzed separately versus 1 liter of PBS, pH 7.4, at 4°C for 8 hrs. After 8 hrs, the used dialyzing PBS was discarded, and fresh PBS was used to continue the dialysis (5 × one liter). After dialysis was complete, the contents of each dialysis membrane was separately centrifuged (15, 600 × G) at 4°C for 6 min. Each supernatant was then stored at 4°C.

### EXAMPLE 2

### Comparison of Haptenylated Streptavidins

Diluted aliquots (1:10) of each of the supernatants were prepared by adding a 100 ul portion of each stored supernatants prepared in Example 1 to a separate 900 ul volume of PBS. Each of the diluted aliquots was read for Optical Density at 424 nm and 280 nm, and the readings and the calculated concentrations of PAN-F reagent moiety (A), streptavidin moiety (B) and mole ratio of PAN-F reagent moiety to streptavidin moiety (A/B) are shown in Table I.

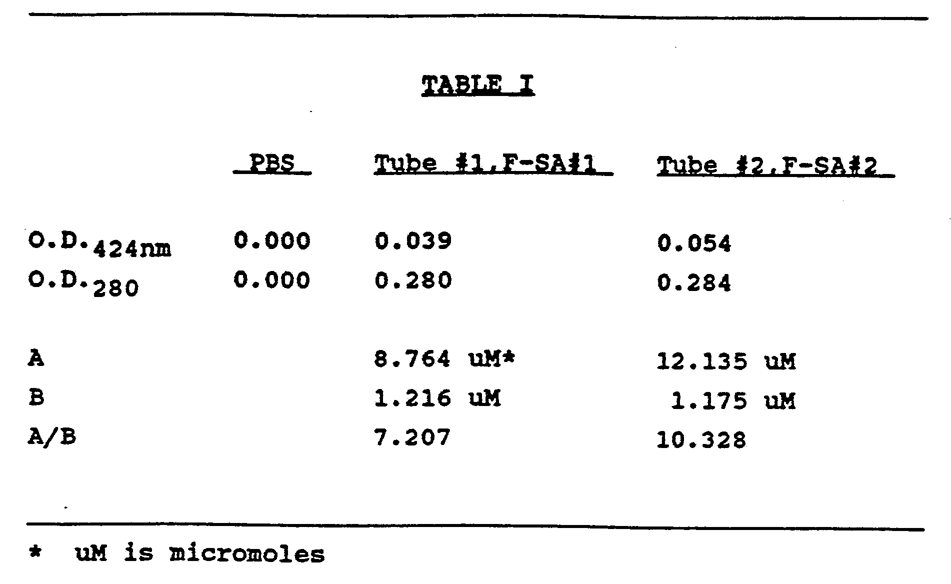

A 96 well microtiter plate was coated with either 200 ul/well of 1 125 ng/ml solution of biotin-BSA (PanAb Laboratories) diluted in Carbonate-Bicarbonate Coating Buffer, pH 9.6, (Na₂CO₃ 1.59 gm/l, NaHCO₃ 2.93 gm/l, NaN₃ .50 gm/l in deionized water) or with 200 ul/well of pure Carbonate-Bicarbonate Coating Buffer, pH 9.6, for 2 hr at 37°C. After 2 hr, the plate was removed from the incubator and washed (3 × 300 ul/well) with TBSX (Tris buffered saline, pH 7.4: Tris-HCl 3.15 g/l, NaCl 8.77 gm/l, Triton X-100 polyoxyethylene ether nonionic surfactant 0.50 gm/l in deionized water). The plate was inverted and allowed to drain on clean absorbent paper for 10 min.

To each well was added 300 ul of BSA-TBSX. BSA-TBSX is 1% BSA in TBSX, pH 7.4. The plate was incubated at 37°C for 2 hr. After 2 hr, the plate was removed from the incubator and washed (3 × 300 ul/well) with TBSX, inverted and allowed to drain on clean absorbent paper for 10 min.

Various weights (1.0, 2.0, 4.0 and 8.0 ng/ml) of either F-SA#1 or F-SA#2 were diluted with BSA-TBSX. 200 ul of each F-SA#1 solution and 200 ul of each F-SA#2 solution were added to individual wells, in duplicate. The microtiter plate was then incubated for 30 min in a 37°C incubator. The plate was then removed from the incubator and washed (3 × 300 ul/well) with TBSX, inverted and allowed to drain on clean absorbent paper for 10 min.

To each well of the microtiter plate was added 200 ul of a 125 ng/ml solution of horseradish peroxidase conjugated with affinity-purified goat IgG antibody specific to PAN-F hapten (PanAb Laboratories) in BSA-TBSX. The microtiter plate was incubated at 37°C for 30 min. The plate was then removed from the incubator and washed (3 × 300 ul/well) with TBSX, inverted and allowed to drain on clean absorbent paper for 10 min.

To each well was added 200 ul of fresh Peroxidase Developer #1 (for 100 ml, 25,7 ml of 0.2 M NaH₂PO₄, 24.3 ml of 0.1 M citric acid, 50.0 ml of deionized water, 40.0 mg of o-phenylenediamine, and 40 ul of 30% H₂O₂). The microtiter plate was incubated in darkness at rm temp for 15 min. The development was stopped by adding 50 ul of 2.5 M H₂SO₄ to each well. The mitrotiter plate was then read for Optical Density of each well using a 96-well visible light microtiter plate reader (MR 580, Dynatech Corporation, Arlington, VA). The results are shown in Table II.

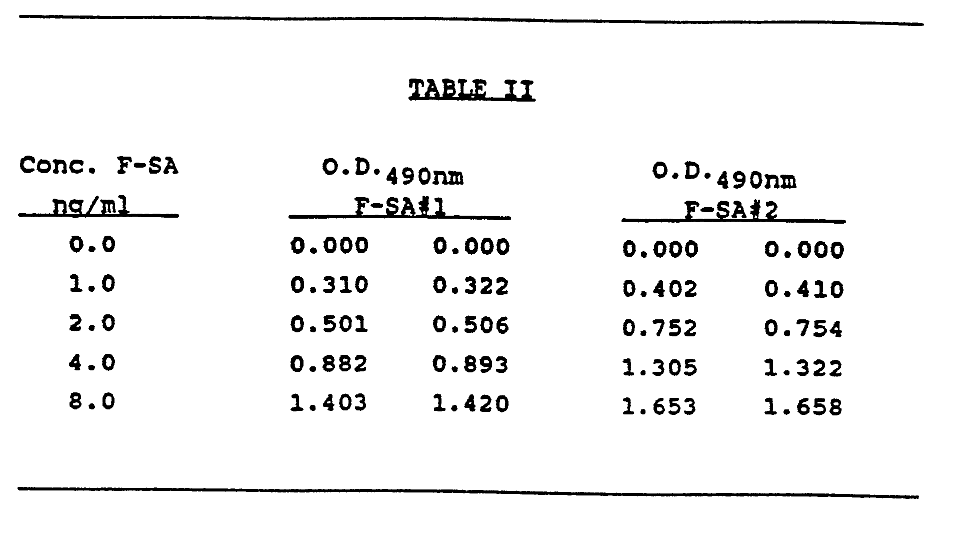

As shown in Table II, even for relatively small differences in the amount of hapten conjugated to steptavidin, there is a significant difference in generated signal. The linear portion of each curve demonstrates an approximate 50% increase in signal for the F-SA#2 preparation over the F-SA#1 preparation. This is approximately the same differential as that found comparing PAN-F/Streptavidin (A/B) in Table I.

### EXAMPLE 3

### Sensitivity Comparison,

### Microtiter Plate

The sensitivity of the haptenylated avidin (PAN-F with streptavidin) system was compared with a direct horseradish peroxidase and with a avidin-biotin system for quantification of murine immunoglobulin G (MIgG).

Microtiter plates were contacted with 200 ul/well of solutions containing different concentrations of MIGG ranging from 0 pg/well (picograms/well) to 10,000 pg/well. The MIgG coating solution concentrations were prepared by dilution with Carbonate-Bicarbonate Coating Buffer described in Example 2, pH 9.6. The solutions were left in the microtiter plate wells for 18 hr at 4°C. Each well was then washed (3 × 300 ul/well) with TBSX described in Example 2, and the plates were inverted and allowed to drain on clean absorbent paper for 10 min. To each well was then added 300 ul/well of BSA-TBSX described in Example 2 and allowed to incubate for 2 hr at 37°C. Each well was then washed (3 × 300 ul/well) with TBSX, and the plates were inverted and allowed to drain on clean absorbent paper for 10 min.

The direct horseradish peroxidase method was conducted as follows. 200 ul of a 1 ug/ml solution of murine anti-IgG antibody conjugated with horseradish peroxidase (HRP-XMIgG) in BSA-TBSX was placed in the microtiter wells of each plate. These wells were allowed to incubate for 30 min at 37°C. Each well was then washed (3 × 300 ul/well) with TBSX, and the plates were inverted and allowed to drain on clean absorbent paper for 10 min. 200 ul/well of Peroxidase Developer #1 described in Example 2 was added and the plates were incubated in darkness for 15 min at rm temp. Further reaction was stopped by adding 50 ul of 2.5 M H₂SO₄ to each well. The plates were immediately read for Optical Density at 490 nm in a visible light microtiter plate reader (MR 580, Dynatech).

The avidin-biotin system method was conducted as follows. 200 ul of a 1 ug/ml solution of biotinylated murine anti-IgG antibody (b-XMIgG) in BSA-TBSX was added to the wells of each plate. The plates were then incubated for 30 min at 37°C. Each well was then washed (3 × 300 ul/well) with TBSX, and the plates were inverted and allowed to drain on clean absorbent paper for 10 min. Then 200 ul/well of a solution of 0.5 micrograms/ml (ug/ml) of horseradish peroxidase conjugated with streptavidin (HRP-SA) in BSA-TBSX was added. The plates were incubated for 30 min at 37°C. Each well was then washed (3 × 300 ul/well) with TBSX, and the plates were inverted and allowed to drain on clean absorbent paper for 10 min. Then 200 ul/well of fresh Peroxidase Developer #1 was added, and the plates were incubated in darkness for 15 min at rm temp. Further reaction was stopped by adding 50 ul of 2.5 M H₂SO₄ to each well. The plates were immediately read for Optical Density at 490 nm in a visible light microtiter plate reader (MR 580, Dynatech).

The haptenylated avidin (PAN-F with steptavidin) method of this invention was carried out as follows. 200 ul of a 1 ug/ml solution of biotinylated murine anti-IgG antibody (b-XMIgG) in BSA-TBSX was added to the wells of each plate. The plates were then incubated for 30 min at 37°C. Each well was then washed ( 3 × 300 ul/well) with TBSX, and the plates were inverted and allowed to drain on clean absorbent paper for 10 min. Then 200 ul/well of a solution of steptavidin conjugated with PAN-F reagent prepared as described in Example 1 was added. The plates were incubated for 30 min at 37°C. Each well was then washed ( 3 × 300 ul/well) with TBSX, and the plates were inverted and allowed to drain on clean absorbent paper for 10 min. Then 200 ul/well of a solution of 0.25 ul/ml of horseradish peroxidase conjugated with anti-(PAN-F) antibody in BSA-TBSX was added. The plates were incubated for 30 min at 37°C. Each well was then washed (3 × 300 ul/well) with TBSX, and the plates were inverted and allowed to drain on clean absorbent paper for 10 min. Then 200 ul/well of fresh Peroxidase Developer #1 was added, and the plates were incubated in darkness for 15 min at rm temp. Further reaction was stopped by adding 50 ul of 2.5 M H₂SO₄ to each well. The plates were immediately read for Optical Density at 490 nm in a visible light microtiter plate reader (MR 580, Dynatech).

Analysis of the results showed that the lower limits of the detection of the murine IgG antibody by the direct method was 200 pg/well, by the avidin-biotin method was 90 pg/well, and by the haptenylated avidin method of this invention was 9 pg/ml.

### EXAMPLE 4

The three detection systems compared in Example 3 were compared by tests performed in a membrane format to determine the maximum sensitivity for quantification of immobilized biotin-bovine serum albumin (b-BSA).

Nitrocellulose membranes (BA-85, Schleicher $ Schuell, Nashua, NH) were spotted with 1 ul of different concentrations of b-BSA. The concentration range of the b-BSA spotted on the membrane was 0 pg/spot to 1000 pg/spot. The membranes were allowed to air dry and were then placed in a plastic weight boat. To this weight boat was added 1 ml of BSA-TBSX per membrane to be blocked. The immersed membranes were shaken gently for 1 hr at rm temp. The membranes were then washed (3 × 2 ml) with TBSX per membrane.

The direct horseradish peroxidase-streptavidin method (HSA-SA) was carried out as follows. The membranes were immersed in a 2.5 ug/ml dilution of horseradish peroxidase conjugated with steptavidin in TSA-TBSX, 1 ml/membrane. The membranes were shaken gently for 30 min at rm temp. The membranes were then washed (3 × 2 ml) with TBSX per membrane. The membranes were developed with fresh Peroxidase Developer #2 (for 20 ml, 0.2 M NaH₂PO₄, 5.1 ml; 0.1 M Citric Acid, 4.9 ml; deionized water, 10.0 ml; 2.5% diaminobenzidene in 10% ethanol, 400.0 ul; 10% CoCl₂ in deionized water, 40.0 ul; and 30% H₂O₂, 40 ul) for 15 min at rm temp. After development, the membranes are washed two times with 2 ml of deionized water per membrane. The membranes are allowed to air dry. Once dry, the membrane is visually examined for spots, and they are graded on a 0 to 4+ scale.

The avidin-biotin method (ABC) was carried out as follows. The membranes were immersed in a 1:1000 dilution of precomplexed streptavidin and b-HRP (Enzo Biochemical, New York, NY) made with BSA-TBSX (1 ml/membrane). The membranes were shaken gently for 30 min at rm temp. The membranes were then washed (3 × 2 ml) with TBSX per membrane. The membranes were developed with fresh Peroxidase Developer #2 (for 20 ml, 0.2 M NaH₂PO₄, 5.1 ml; 0.1 M Citric Acid, 4.9 ml; deionized water, 10.0 ml; 2.5% diaminobenzidene in 10% ethanol, 400.0 ul; 10 % CoCl₂ in deionized water, 40.0 ul; and 30% H₂O₂, 40 ul) for 15 min at rm temp. After development, the membranes are washed two times with 2 ml of deionized water per membrane. The membranes are allowed to air dry. Once dry, the membrane was visually examined for spots, and the spots were graded on a 0 to 4+ scale.

The haptenylated avidin method of this invention (HAPVIDIN) was carried out as follows. The membranes were immersed in a 2.5 ug/ml solution of streptavidin conjugated with PAN-F reagent prepared as described in Example 1 made up in BSA-TBSX (1 ml/membrane). The membranes are shaken gently for 30 min at rm temp. The membranes are then washed (3 × 2 ml/membrane) with TBSX. The membranes are then immersed in a 2.5 ug/ml solution of horseradish peroxidase conjugated with anti-PAN-F antibody (1 ml/membrane). The membranes were shaken gently for 30 min at rm temp. The membranes were then washed (3 × 2 ml) with TBSX per membrane. The membranes were developed with fresh Peroxidase Developer #2 (for 20 ml, 0.2 M NaH₂PO₄ 5.1 ml; 0.1 M Citric Acid, 4.9 ml; deionized water, 10.0 ml; 2.5% diaminobenzidene in 10% ethanol, 400.0 ul; 10% CoCl₂ in deionized water, 40.0 ul; and 30% H₂O₂, 40 ul) for 15 min at tm temp. After development, the membranes are washed two times with 2 ml of deionized water per membrane. The membranes are allowed to air dry. Once dry, the membrane was visually examined for spots, and the spots were graded on a 0 to 4+ scale.

The results are shown in Table III

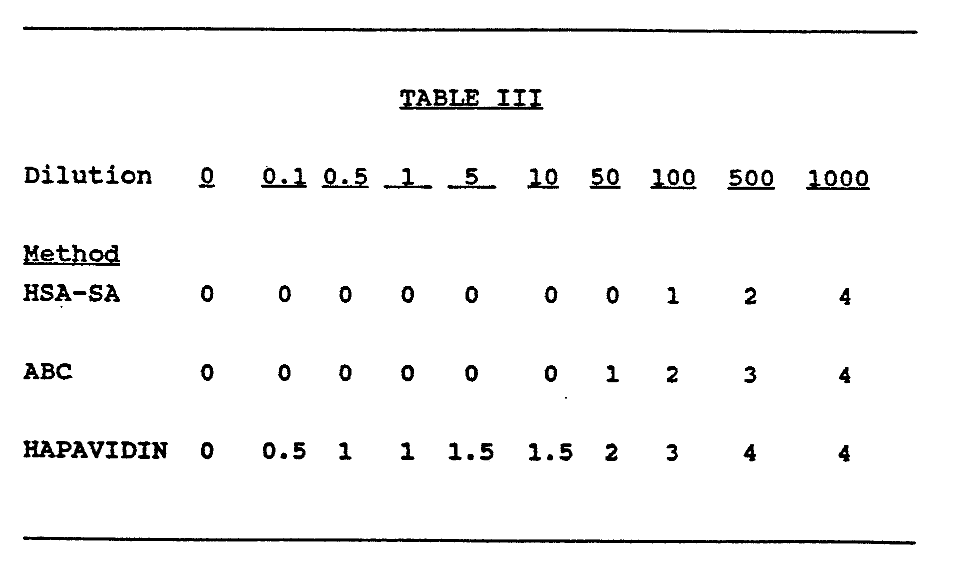

As shown in Table III, the lower limits of detection for spotted b-BSA were 100 pg b-BSA/spot for the direct method HRP-SA, 50 pg b-BSA/spot for the avidin-biotin method ABC, and 0.1 b-BSA/spot for the method of this invention (HAPAVIDIN).

### EXAMPLE 5

### Haptenylated Avidin

To 1.0 ml of carbonate buffer solution, pH 9.0 having avidin (2 mg) dissolved therein is added 4-fluoro-3-nitro-N,N,N-trimethylammoniumbenzene iodide (2 mg). The solution is incubated at 37°C for 16 hr, and the reaction is terminated by adding 1.0 ml of glycine (10 mg/ml, pH 7.0). The solution is dialyzed exhaustively against phosphate buffered saline (PBS), pH 7.1, to remove low molecular weight materials. The solution contains 3-nitro-N,N,N-trimethylammonium-4-phenyl substituted avidin.

### EXAMPLE 6

### Haptenylated Avidin

To 1.0 ml of carbonate buffer solution, pH 9.0, having avidin (2 mg) dissolved therein is added 2-chloro-3,5-dinitropyridine (2 mg). The solution is incubated at 37°C for 3 hr, and the reaction is terminated by adding 1.0 ml of glycine (10 mg/ml, pH 7.0. The solution is dialyzed exhaustively against phosphate buffered saline (PBS), pH 7.2, to remove low molecular weight materials. The solution contains 3,5-dinitro-2-pyridyl substituted avidin.

### EXAMPLE 7

### Other Haptenylated Avidins

Repeating the procedure of Example 5 but replacing the 4-fluoro-3-nitro-N,N,N-trimethylammoniumbenzene iodide with 4-fluoro-N,N,N-trimethylammonium benzene iodide, 4-fluoro-2-nitro-N,N,N-trimethylammoniumbenzene iodide,
4-fluoro-3,5-dinitro-N,N,N-trimethylammoniumbenzene iodide, 3-fluoro-4-nitro-N,N,N-trimethylammoniumbenzene iodide, 3-fluoro-N,N,N-trimethylammoniumbenzene iodide, 4-chloro-N,N,N-trimethylammoniumbenzene iodide, 4-chloro-3-nitro-N,N,N-trimethylammoniumbenzene iodide, 4-chloro-2-nitro-N,N,N-trimethyalmmoniumbenzene iodide, 4-chloro-3,5-dinitro-N,N,N-trimethylammoniumbenzene iodide, 3-chloro-4-nitro-N,N,N-trimethylammoniumbenzene iodide, N,N,N-trimethylammoniumbenzene-4-sulfonic acid iodide;
3-nitro-N,N,N-trimethylammoniumbenzene-4-sulfonic acid iodide, 2-nitro-N,N,N-trimethylammoniumbenzene-4-sulfonic acid iodide,
3,5-dinitro-N,N,N-trimethylammoniumbenzene-4-sulfonic acid iodide,
4-nitro-N,N,N-trimethylammoniumbenzene-3-sulfonic acid iodide, yields the corresponding N,N,N-trimethylammonium-4-phenyl substituted avidins.

### EXAMPLE 8

### Haptenylated KLH Antigen

To 5.0 ml of PBS solution, pH 9.0, having KLH antigen (9 mg/ml) dissolved therein is added 1.6 ml of 0.2 M carbonate buffer (pH 9.0) and 2.4 ml of 0.01 M 4-fluoro-3-nitro-N,N,N-trimethylammoniumbenzene iodide in carbonate buffer. This is incubated overnight at 37°C and repeatedly dialyzed against PBS to remove low molecular weight materials. The solution contains 3-nitro-N,N,N-trimethylammonium-4-phenyl substituted KLH antigen.

### EXAMPLE 9

### AntiHapten Polyclonal Antibodies

Antibodies which specifically couple with the 3-nitro-N,N,N-trimethylammonium-4-phenyl group are isolated from the serum of a goat immunized with the amine-group linked 3-nitro-N,N,N-trimethylammonium-4-phenyl substituted KLH antigen prepared in accordance with the procedure of Example 8 in accordance with procedures described by Fenton and Singer, Biochemistry. 10:1429-1473 (1971). The antisera is passed over a column of agarose beads to which 3-nitro-N,N,N-trimethylammonium-4-phenyl groups have been attached by BSA. The BSA is attached to beaded agarose after activation of the agarose with cyanogen bromide at pH 5.5. The 3-nitro-N,N,N-trimethylammonium-4-phenyl groups are attached to the spacer groups by reacting 4-fluoro-3-nitro-N,N,N-trimethylamoniumbenzene iodide therewith under the reaction conditions set forth in Example 5.

The 3-nitro-N,N,N-trimethylammonium-4-phenyl specific antibody is eluted by passage of 3-nitro-N,N,N-trimethylammonium-4-phenyl substituted amine groups over the column. The antibody-containing solution is dialyzed against PBS, pH 7.2, to free it from the substituted amine groups.

### EXAMPLE 10

### Haptenylated KLH Antigen

To 1.0 ml of carbonate buffer solution, pH 9.0, having KLH antigen (2 mg) dissolved therein is added 2-chloro-3,5-dinitropyridine (2 mg). The solution is incubated at 37°C for 3 hr, and the reaction is terminated by adding 1.0 ml of P-glycine (10 mg/ml, pH 7.0). The solution is dialyzed exhaustively against phosphate buffered saline (PBS), pH 7.2, to remove low molecular weight materials. The solution contains 3,5-dinitro-2-pyridyl substituted KLH antigen.

### EXAMPLE 11

### Other AntiHapten Polyclonal Antibodies

Antibodies which specifically couple with the 3,5-dinitro-2-pyridyl group are isolated from the serum of a goat immunized with the amine-group linked 3,5-dinitro-2-pyridyl substituted KLH antigen prepared in accordance with the procedure of Example 10 in accordance with procedures described by Fenton and Singer, Biochemistry. 10:429-1473 (1971). The antisera is passed over a column of agarose beads to which 3,5-dinitro-2-pyridyl groups have been attached by BSA. The BSA is attached to beaded agarose after activation of the agarose with cyanogen bromide at pH 5.5. The 3,5-dinitro-2-pyridyl groups are attached to the spacer groups by reacting 2-chloro-3,5-dinitro-2-pyridine therewith under the reaction conditions set forth in Example 6.

The 3,5-dinitro-2-pyridyl specific antibody is eluted by passage of 3,5-dinitro-2-pyridyl substituted amine groups over the column. The antibody-containing solution is dialyzed against PBS, pH 7.2, to free it from the substituted amine groups.

### EXAMPLE 12

### Horse Radish Peroxidase labeled antibodies

Conjugates of horse radish peroxidase (HRP, Sigma) with goat anti-(3-nitro-N,N,N-trimethylammonium--4-phenyl) antibody are prepared by the method of Iskikawa, E. Journal of Immunoassay. 4(3) (1983). Malemide groups are introduced into the enzyme by treatment with N-succinimidyl-4-(N-maleimidomethyl)-cyclohexane-1-carboxylate. Thiol groups are attached to the antibody by reaction with S-acetyl-mercaptosuccinic anhydride. The maleimide-enzyme and mercaptosuccinylated antibody are then reacted to produce the conjugate. Conjugates are purified by gel filtration chromatography on a Fast Protein Liquid Chromatogrphy System (FPLC).

### EXAMPLE 13

### Other enzyme labeled antibodys

Conjugates of horse radish peroxidase (HRP, Sigma) with goat anti-(3,5-dinitro-2-pyridyl) antibody are prepared by the method of Iskikawa, E. Journal of Immunoassay. 4(3) (1983) as described in Example 12.

Conjugates of alkaline phosphatase with goat anti-(3-nitro-N,N,N-trimethylammonium-4-phenyl) antibody and with goat anti-(3,5-dinitro-2-pyridyl) antibody are prepared by the same procedure.

### EXAMPLE 14

### Mouse monoclonal antibody assay

Wells of microtiter plates are coated with goat anti-mouse IgG antibodies (Capell Laboratories) by incubation at 4°C for 16 hr, each well containing 200ul of antibody (25 microgram/ml in 0.1 M NaHCO₃, pH 9.5). The plates are then washed 4 times with PBS containing 0.05% non-ionic surfactant (TWEEN 20), pH 7.2. The plates are then incubated with PBS containing 1% BSA and 0.5% TWEEN 20 for 2 hours at 37°C. To quantify the amount of IgG in hybridoma supernatants, 100 microliters of normal mouse IgG or supernatant suitably diluted in PBS containing 1% BSA is placed in each well and incubated at 37°C for 2 hours. The well contents are discarded, and the plates are washed 4 times with PBS containing 0.05% TWEEN 20.

To each well of the plate, 200 microliters (0.2 ug/ml in PBS containing 1% BSA and 0.05% TWEEN 20) of biotin-labeled goat anti-mouse antibody (Sigma) is added, and the plates are incubated at 37°C for 2 hrs. The plates are then washed 4 times with PBS containing 0.05% TWEEN 20, pH 7.2. Each well then receives 200 microliters of solution containing 3-nitro-N,N,N-trimethylammonium-4-phenyl coupled avidin (1.0 microliters/ml in PBS) prepared in accordance with the procedure of Example 5 and horseradish peroxidase-conjugated goat anti-(3-nitro-N,N,N-trimethylammonium-4-phenyl) antibodies prepared in accordance with the procedure of Example 11 (0.125 micrograms/ml in PBS containing 1% BSA, 0.05% TWEEN, pH 7.2). This solution is incubated in the wells for 30 min at 37°C and then discarded. The plates are then washed 4 times with PBS containing 0.05% TWEEN 20, pH 7.5.

The wells are then filled with 200 microliters of a substrate solution consisting of 10 mg o-phenylendediamine dihydrochloride and 10 microliters of 30% hydrogen peroxide in 25 ml of disodium phosphate-citrate buffered solution, pH 6.5. After incubating for 30 min, the substrate reaction is stopped by adding 50 microliters/well of 2.5 M H₂SO₄. The plates are read immediately at 492 nm.

### EXAMPLE 15

### Lawn method

For purposes of screening cDNA or genomic libraries for antigen expression by recombinant clones, E. coli Y1090 is grown in the presence of lambda gt 11 phage clones which will cause the expression of ovalbumin. The materials are obtained from Clontech Laboratories, 4055 Fabian Way, Palo Alto, CA 94304. The E. coli is first streaked on LB plates containing 50 microliters/ml ampicillin. From these, single colonies are isolated and then grown to saturation in LB broth containing 2% maltose. The 0.2 ml of the culture is mixed with 0.1 ml of sterile diluent containing the lambda gt 11 phage and/or the c1 clone containing the ovalbumin gene for expression. After 15 min, this is mixed with LB soft agar and poured onto an LB plate. Plates are incubated at 42.5°C for 3.5 hr.

Nitrocellulose filters are saturated in 10 mM isopropyl β-D-thiogalactopyranoside and thoroughly dried. A dried filter is placed on the surface of the culture plate and allowed to remain in contact for 3.5 hr at 37°C. Filters are then rinsed in a solution (TBS) containing 50 mM Tris (pH 7.9) and 150 mM NaCl, to which 0.05% TWEEN 20 has been added.

The filters are then incubated in a solution having this composition and in addition, 20% fetal calf serum, for 30 min at room temperature and then rinsed. The filters are then incubated with rabbit anti-ovalbumin serum for one hr. After washing, the filters are placed in a solution containing biotinylated goat anti-rabbit antibody and incubated at room temperature for 30 min. The filters are then washed in TBS containing 0.05% TWEEN 20 3 times, allowing 3 min per change. The filters are then transferred to a solution containing 3-nitro-N,N,N-trimethylammonium-4-phenyl substituted avidin, and the corresponding anti-(3-nitro-N,N,N-trimethylammonium-4-phenyl) antibody labeled with horseradish peroxidase. This solution is composed of 0.3 micrograms/ml of the 3-nitro-N,N,N-trimethylammonium-4-phenyl substituted avidin prepared in accordance with Example 5 and a 1/50 dilution of 50 micrograms/ml of the horseradish peroxidase labeled antibody prepared in accordance with the procedure of Example 14 in 5 ml of TBS containing 0.05% TWEEN and 0.1% BSA. pH 7.5. Before using the mixture, it is permitted to sit for 30 min at room temperature.

Filters are immersed in the solution for 30 min at room temperature, and are then washed in TBS 3 times, allowing 3 min per wash.

Filters are then transferred to a peroxidase substrate solution and allowed to develop for 30 min. They are then washed 3 times in distilled water and air dried.

The substrate solution is prepared by mixing 2 ml of 4-chloro-1-naphthol (3 mg/ml in methanol) with 10 ml of TBS which contains 0.01 M imidazole. Immediately before use, 5 microliters of 30% hydrogen peroxide is added to the solution.

### EXAMPLE 16

### Blot method

Dot and Southern blots are prepared and probed as described by Leary et al. Proc.Natl.Acad.Sci.USA. 80:4045-4049 (1983) using biotinylated probes prepared with 5-(N-(N-biotinylated-epsilon-aminocaproyl)-3-aminoallyl)-deoxyuridine triphosphate, Bio-11-dUTP. Following the hybridization, the filters are washed, dried and rehydrated in a 0.1 M TRIS buffer, pH 7.5, containing 3% BSA for 30 min at 42°C. The filters are then baked at 80°C, followed again by rehydration in the TRIS-BSA solution.

The filters are then immersed in a solution containing 0.3 micrograms/ml of the 3-nitro-N,N,N-trimethylammonium-4-phenyl substituted avidin prepared in accordance with the procedure of Example 5 and a 1/550 dilution of 50 micrograms/ml of alkaline phosphatase labeled goat anti-(3-nitro-N,N,N-trimethylammonium-4-phenyl) antibody prepared in accordance with the procedure of Example 12, dissolved in the same TRIS solution containing 0.05% TWEEN AND 0.1% BSA, pH 7.5. This solution is allowed to sit for 0.5 hr before use.

After immersion in the avidin-antibody solution for 0.5 hr, the filters are washed 3 times in TRIS, pH 7.5, and then twice in a solution containing 0.1 M TRIS, pH 9.5, 0.1 M NaCl and 5 mM Magnesium chloride. Color development is then carried out using nitroblue tetrazolium 5-bromo-4-chloro-3-indolyl phosphate substrate solution described by Leary, supra. Development is terminated and the filters are washed and dried, following the Leary protocol.

### EXAMPLE 17

Polystyrene microtiter plate wells are coated with rabbit IgG specific for human IgG. To 500 microliters of dilutions of human serum samples, an equal volume of biotinylated human IgG is added in a plain test tube and mixed. Aliquots (100 microliters/well) of the solution is then transferred to the coated microtiter plate wells and allowed to sit for 90 min. The wells are then drained and washed 3 times with PBS - TWEEN 20 rinse solution.

A dilution of 3-nitro-N,N,N-trimethylammonium-4-phenyl substituted avidin is then added to the wells and allowed to sit for 30 min. The wells are again drained and washed 3 times with the rinse solution.

Anti-(3-nitro-N,N,N-trimethylammonium-4-phenyl) antibody labeled with horseradish peroxidase prepared in accordance with the procedure of Example 12 (100 microliters/well) is then added to the wells and allowed to sit for 30 min. The wells are then drained and washed 3 times with the rinse solution.

The wells are then filled with 200 microliters of a substrate solution consisting of 10 mg o-phenylenediamine dihydrochloride and 10 microliters of 30% hydrogen peroxide in 25 ml of disodium phosphate-citrate buffered solution, pH 6.5. After incubating for 30 min, the substrate reaction is stopped by adding 50 microliters/well of 2.5 M H₂SO₄. The plates are read immediately at 492 nm.

### EXAMPLE 18

### Hapten-Avidin Conjugate

Following the procedure described in U.S. Patent 3,825,525, 50 mg of 4-amino-3-nitro-1-(N,N,N-trimethylammonium)benzene is dissolved in 5 ml of 0.5 M borate fuffer, pH 8.85. 5 ml of a 2% solution of 1-cyclohexyl-3-(2-morpholinoethyl)carbodiimide metho-p-toluenesulphonate is added slowly. The solution is stirred at room temperature overnight to yield the hapten-avidin conjugate.

### EXAMPLE 19

### HYBRIDIZATION OF FILTER BOUND DNA

### 1) Prehybridization

Wet nitrocellulose filters evenly, floating them on a solution of 1% TRITON X-100. Blot the filters gently on WHATMAN 3MM paper to remove excess liquid. The pre-hybridization and hybridization reactions are carried out in a polyethylene bag (SEARS SEAL-N-SAVE BOILABLE COOKING POUCHES OR LAYFLAT POLYTHENE TUBING (TRANS-ATLANTIC SUPPLIES). Place each filter in a separate bag and heat-seal this, except for one corner, using a domestic bag sealer.

A pre-hybridization buffer solution containing formamide is prepared by mixing a first solution containing 50 ml deionized formamide, 25 ml of SSC (0.15 M NaCl, 0.15 M trisodium citrate, pH 7.0), 5 ml of Denhardt's solution (2% Ficoll, mol. wt. 400,000; 2% polyvinyl pyrrolidone, mol. wt. 400,000; 2% BSA) and 5 ml of sodium phosphate buffer, pH 6.8, 0.5% SDS, adjusted to 95.5 ml with distilled water. With this is mixed a second solution comprising 2 ml of sonicated calf thymus DNA, 0.2 ml of Poly(C) (5 mg/ml), 0.2 ml of poly(A) (5 mg/ml), 2 ml of yeast tRNA (5 mg/ml) which has been premixed, denatured in a boiling water bath for 5 min and quenched with ice.

Add the pre-hybridization solution to the bag (0.08 ml/cm² of filter), pre-warmed to 42°C, squeezing out the air bubbles and sealing the corner. The bag is incubated for 4-24 hr at 42°C by placing it in a box of water at 42°C in a shaking water bath at the same temperature. Cut the corner of the bag and drain the liquid out, removing as much liquid as possible but not allowing the filter to dry out.

### 2) Hybridization

The hybridization is carried out in a standard hybridization solution containing formamide. The is prepared from a first solution prepared by mixing together 50 ml of deionized formamide, 25 ml of SSC, 1 ml of Denhardt's solution, 2 ml of 1 M sodium phosphate buffer, pH 6.8, 1 ml of 20% SDS, and 10 g of dextran sulfate (mol. wt. 500,000), stirred until the dextran sulfate is dissolved and adjusting the volume to 95.5 ml with distilled water. With this is mixed a second solution prepared by mixing together 2 ml of sonicated DNA (5 mg/ml), 0.2 ml of poly(C) (5 mg/ml), 0.2 ml of poly(A) (5 mg/ml) and 2 ml of yeast tRNA (5 mg/ml), denatured in a boiling water bath for 5 min, and quenched in ice.

The hybridization buffer is prewarmed, and biotinylated probe prepared in accordance with the procedure of European Patent Application 155,854 from photobiotin is added to a concentration which does not exceed 10 ng probe/ml. Immediately add this solution to the filter (0.05ml/cm²) and reseal the bag. Hybridize the filter at 42°C for between 6 and 48 hr. A corner of the bag is cut and the hybridization solution is removed. The bag is then cut open completely and the filter is removed and immersed in 200 ml of 2 × SSC, 0.1% SDS at rm temp, shaking the filter gently, and rinsing the filter twice for 5 min each with this solution. For a moderately stringent wash, wash the filter twice in 400 ml of 2 × SSC,. 0.1% SDS at 60°C for 1 hr. For a higher stringency wash, treat the filter for 2 × 1 hr at 65°C in 0.1 × SSC, 0.1% SDS. Finally rinse the filter in 2 × SSC at rm temp. Blot the filter to remove excess liquid. Finally, rinse the filter in 2 × SSC at rm temp, blot the filter to remove excess liquid.

### 3) Label determination

The filter is immersed in a solution containing 3-dinitro-N,N,N-trimethylammonium-4-phenyl coupled avidin (1.0 micrograms/ml in PBS) and horseradish peroxidase conjugated goat anti-TMP antibody (0.125 micrograms/ml in PBS containing 1% BSA, 0.05% TWEEN-20, pH 7.2. This solution is incubated for 30 min at 37°C and then discarded. The filter is then washed 4 times with PBS containing 0.05% TWEEN-20, pH 7.5.

The filter is then immersed in a substrate solution containing 10 mg of o-phenylenediamine dihydrochloride and 10 microliters of 30% hydrogen peroxide in 25 ml of disodium phosphate-citrate buffered solution, pH 6.5. After incubating for 30 min, the substrate solution is stopped by adding 2.5 M H₂SO₄. The filter is then examined immediately for fluorescence at 492 nm.

### EXAMPLE 20

### In situ hybridization

### 1) Sample preparation and fixation

Fetal cells obtained from an amniocentesis sample are plated on coverslips which have been acid washed (boiled in 0.1 M HCl) and autoclaved in 0.5% gelatin and cultured. The coverslips are rinsed in Hanks Balanced Salt Solution, and then fixed in 4% paraformaldehyde in phosphate buffered saline (PBS) and 5 mM MgCl₂, pH 7.4 for 15 min. The fixative solution is made by dissolving paraformaldehyde in PBS and sitting on low heat for 2-4 hr. After the paraformaldehyde dissolves, the magnesium chloride is added, and the mixture filtered.

### 2) Hybridization

The cells are rehydrated in PBS and 5 mM MgCl₂ for 10 min, using small Coplin jars. They are then transferred to 0.2 M Tris-HCl, pH 7.4: 0.1 M glycine for 10 min. They are then transferred to 50% deionized formamide (FLUKA), 2 × SSC. Just prior to hybridization, heat at 65°C for 10 min. The formamide is deionized by stirring with mixed bed resin (AG501-XB, BIORAD #142-6425) for 0.5 hr, and then filtering with WHATMAN #1 paper. The 20 × SSC is 3 M NaCl, 0.3 M sodium citrate, pH 7.2.

20 Micrograms of biotinylated probe prepared from biotinylated adenine and cytosine bases thereof is dried down in a microcentrifuge tube with 20 micrograms of tRNA (E. coli) and 20 micrograms of sheared salmon sperm DNA. In the microcentrifuge tube, melt probe in 100% deionized formamide (10 microliters/sample) for 10 min at 90°C. Immediately before hybridization, add the following to the microcentrifuge tube containing the probe and formamide: 2 microliters of 20 × SSC, 2 microliters of 2% BSA, 4 microliters of 50% dextran sulfate in water and 2 microliters of vanadyl-sulfate ribonucleotide complex RNAse inhibitor. This can be made as a mix before adding to the 90°C formamide. 20 Microliters of the hot hybridization mix is applied directly on parafilm affixed to a glass plate, and the coverslip cell slide is placed down on the drop. The samples are covered with parafilm and incubated in a humidified incubator at 37°C for 4 hr.

### 3) Washing

The samples are removed and washed in 50% formamide, 2 × SSC for 30 min at 37°C. The wash is repeated in 50% formamide, 1 × SSC for 30 min at 37°C. The wash is then repeated in 1 × SSC at room temperature with gentle agitation for 30 min with several changes of 1 × SSC.

### 4) Detection

After the hybridization wash, the coverslips are incubated at 42°C for 10 min in a rinse solution containing 0.1 M Tris-HCl, 0.1 M NaCl, 2 mM MgCl₂, 0.5% TRITON X-100 and 3% BSA. They are then incubated with 0.3 micrograms/ml of the 3-nitro-N,N,N-trimethylammonium-4-phenyl substituted avidin prepared in accordance with the procedure of Example 5 and a 1/550 dilution of 50 micrograms/ml of alkaline phosphatase labeled goat anti-(3-nitro-N,N,N-trimethylammonium-4-phenyl) antibody prepared in accordance with the procedure of Example 12, dissolved in the same TRIS solution containing 0.05% TWEEN AND 0.1% BSA, pH 7.5. This solution is allowed to sit for 0.5 hr before use.

After immersion in the avidin-antibody solution for 0.5 hr, the coverslips are washed 3 times in TRIS, pH 7.5, and then twice in a solution containing 0.1 M TRIS, pH 9.5, 0.1 M NaCl and 5 mM Magnesium chloride. Color development is then carried out using nitroblue tetrazolium 5-bromo-4-chloro-3-indolyl phosphate substrate solution described by Leary, supra. Development is terminated and the coverslips are washed and dried, following the Leary protocol.

## Claims

1. A water-soluble avidin derivative of the formula:
(Hapten)ₐ-A (I)
wherein
Hapten is a water-soluble, non-antigenic group having a molecular weight of less than 5000,
A is avidin,
a is from 1 to 40 inclusive, and
wherein Hapten is covalently bonded to A through an amino or carboxyl group thereof or through an azo linkage with a phenol group of a tyrosine or an imidazole group of a histidine group thereof.

2. A water-soluble avidin derivative of Claim 1 of the formula: 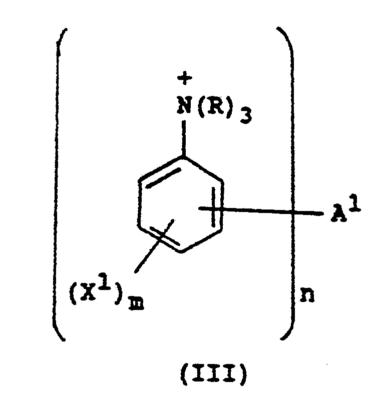 wherein
A¹ is avidin bonded through an amino or carboxyl linkage thereof or an azo linkage with a phenol group of a tyrosine or an imidazole group of a histidine group thereof;
R is methyl or ethyl;
X¹ is nitro, halo, methoxy, carboxy or acetyl;
m is 0, 1 or 2; and
n is from 1 to 40.

3. As a water-soluble avidin derivative of Claim 2, 3-nitro-N,N,N-trimethylammonium-4-phenyl substituted avidin.

4. A water-soluble avidin derivative of Claim 1 of the formula: 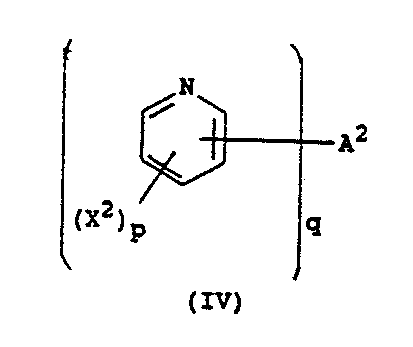 wherein
A² is avidin bonded through an amino or carboxyl linkage thereof, or an azo group with a phenol group of a tyrosine or an imidazole group of a histidine group thereof;
X² is nitro, halo, methoxy, carboxy or acetyl;
p is 1 or 2; and
q is from 1 to 40.

5. As a water-soluble avidin derivative of Claim 4, 3,5-dinitro-2-pyridyl substituted avidin.

6. A water-soluble avidin derivative of Claim 1 of the formula: 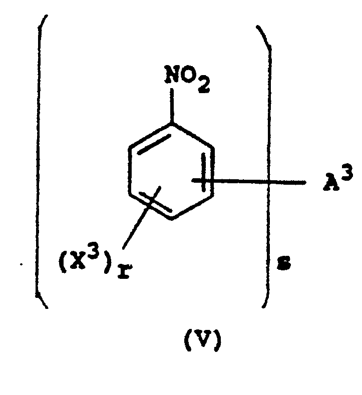 wherein
A³ is avidin bonded through an amino or carboxyl linkage thereof, or through an azo linkage with a phenol group of a tyrosine or an imidazole group of a histidine group thereof;
X³ is nitro, halo, methoxy, carboxy or acetyl;
r is 1 or 2; and
s is from 1 to 40.

7. As an avidin derivative of Claim 6, 1,5-dinitro-3-phenyl substituted avidin.

8. A water-soluble avidin derivative of Claim 1 of the formula: 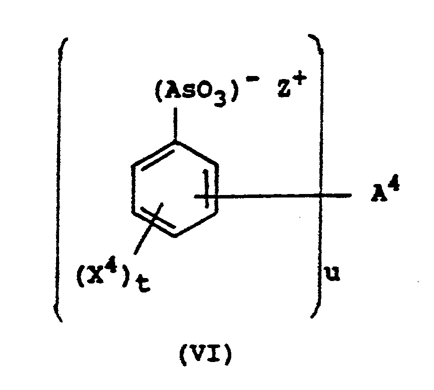 wherein
A⁴ is avidin bonded through an azo linkage with the phenol group of a tyrosine or the imidazole group of a histidine group thereof;
X⁴ is nitro, halo, methoxy, carboxy or acetyl;
Z is an alkali metal, alkaline earth metal, ammonium group, or quaternary lower alkyl amino group;
t is 0, 1 or 2; and
u is from 1 to 40.

9. As an avidin derivative of Claim 8, azobenzenearsonate substituted avidin.

10. An immunoassay comprising the steps of
(a) contacting a water-soluble avidin derivative of Formula I with a labeled antHapten antibody for a time sufficient to permit Hapten-antibody conjugation:
(Hapten)ₐ-A (I)
wherein
Hapten is water-soluble, non-antigenic group having a molecular weight of less than 5000
A is avidin,
a is from 1 to 40, and
wherein Hapten is covalently bonded to A through an amino or carboxyl group thereof or through an azo group linkage with a phenol group of a tyrosine or an imidazole group of a histidine group thereof; and
(b) determining the label present in the labeled antiHapten antibody conjugated with Hapten or in the unconjugated antiHapten antibody.

11. The immunoassay of Claim 10, wherein the water-soluble avidin derivative is of the formula: 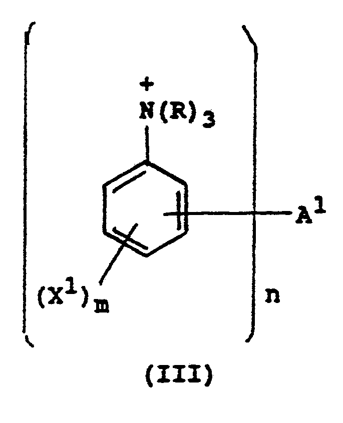 wherein
A¹ is avidin bonded through an amino or carboxyl linkage thereof or an azo linkage with a phenol group of a tyrosine or an imidazole group of a histidine thereof;
R is methyl or ethyl;
X¹ is nitro, halo, methoxy, carboxy or acetyl;
m is 0, 1 or 2; and
n is from 1 to 40.

12. The immunoassay of Claim 11, wherein the water-soluble avidin derivative is 3-nitro-N,N,N-trimethylammonium-4-phenyl substituted avidin.

13. The immunoassay of Claim 10, wherein the water-soluble avidin derivative is of the formula: 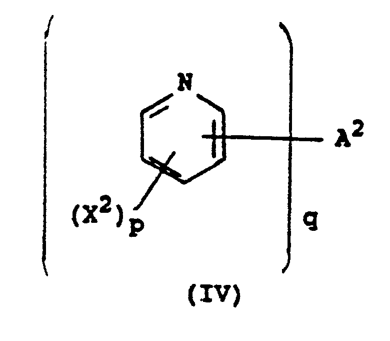 wherein
A² is avidin bonded through an amino or carboxyl linkage thereof, or an azo group with a phenol group of tyrosine or an imidazole group of a histidine group thereof;
X² is nitro, halo, methoxy, carboxy or acetyl;
p is 1 or 2; and
q is from 1 to 40.

14. The immunoassay of Claim 13, wherein the water-soluble avidin derivative is 3,5-dinitro-2-pyridyl substituted avidin.

15. The immunoassay of Claim 10, wherein the water-soluble avidin derivative is of the formula: 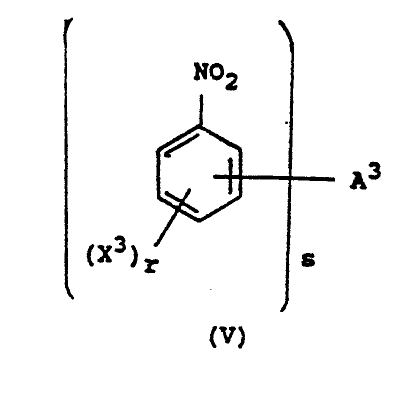 wherein
A³ is avidin bonded through an amino or carboxyl linkage thereof, or through an azo linkage with a phenol group of a tyrosine or an imidazole group of a histidine group thereof;
X³ is nitro, halo, methoxy, carboxy or acetyl;
r is 1 or 2; and
s is from 1 to 40.

16. The immunoassay of Claim 15, wherein the water-soluble avidin derivative is 1,5-dinitro-3-phenyl substituted avidin.

17. The immunoassay of Claim 10, wherein the water-soluble avidin derivative is of the formula: 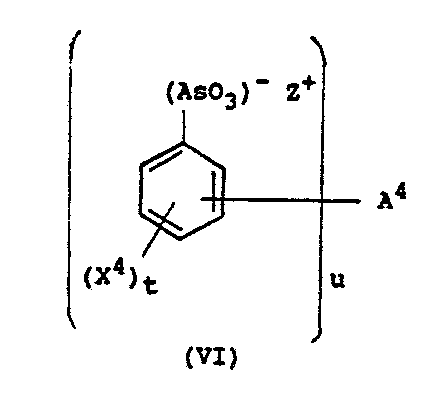 wherein
A⁴ is avidin bonded through an azo linkage with the phenol group of a tyrosine or the imidazole group of a histidine group thereof;
X⁴ is nitro, halo, methoxy, carboxy or acetyl;
Z is an alkali metal, alkaline earth metal, ammonium group, or quaternary lower alkyl amino group;
t is 0, 1 or 2; and
u is from 1 to 40.

18. The method of Claim 17, wherein the water-soluble avidin derivative is azobenzenearsonate substituted avidin.

19. An immunoassay of Claim 10, wherein the avidin derivative of Formula I is bound to a biotinylated reagent.

20. An immunoassay of Claim 19, wherein the avidin derivative of Formula I is bound to a biotinylated nucleic acid probe.

21. An immunoassay of Claim 19, wherein the avidin derivative of Formula I is bound to a biotinylated antibody.

22. An immunoassay kit comprising the avidin derivative of any one of Claims 1 to 9 and a biotinylated reagent.

23. The immunoassay kit of Claim 22, wherein the biotinylated reagent is a biotinylated nucleic acid probe.

24. The immunoassay kit of Claim 22, wherein the biotinylated reagent is a biotinylated antibody.

25. An immunoassay kit of Claim 22 and an antibody which binds specifically with Hapten.

26. An immunoassay kit comprising the avidin derivative of any one of claims 1 to 9 and an antibody which binds specifically with Hapten.
